# EUROPÄISCHE PATENTSCHRIFT

(11) **EP 1 082 449 B1**
(45) Veröffentlichungstag und Bekanntmachung des Hinweises auf die Patenterteilung: **05.03.2003**
(21) Anmeldenummer: 99955299.5
(22) Anmeldetag: 20.05.1999
(51) Int. Cl.: C12P 13/00, C12P 41/00

(54) **VERFAHREN ZUR STEREOSELEKTIVEN HERSTELLUNG VON SUBSTITUIERTEN CYCLOHEXYLCYANHYDRINEN**
METHOD FOR STEREOSELECTIVE PRODUCTION OF SUBSTITUTED CYCLOHEXYLCYANHYDRINS
PROCEDE DE PRODUCTION STEREOSELECTIVE DE CYCLOHEXYLCYANHYDRINES SUBSTITUEES

(30) Priorität: 02.06.1998 DE 19824491
(43) Veröffentlichungstag der Anmeldung: 14.03.2001
(73) Patentinhaber: Bayer CropScience AG, 40789 Monheim (DE)
(72) Erfinder: EFFENBERGER, Franz, D-70597 Stuttgart (DE); ROOS, Jürgen, D-70193 Stuttgart (DE); FISCHER, Reiner, D-40789 Monheim (DE)
(86) Internationale Anmeldenummer: EP9903464
(87) Internationale Veröffentlichungsnummer: WO99063104

(56) Entgegenhaltungen:
- EP-A2- 0 799 894
- WO-A1-93/11255
- DE-C1- 4 102 327

## Beschreibung

Die vorliegende Erfindung betrifft ein neues Verfahren zur stereoselektiven Herstellung von substituierten Cyclohexylcyanhydrinen.

Es ist bekannt, daß sich Cyanhydrine durch Umsetzung von Ketonen mit Blausäure herstellen lassen (siehe z.B. Beyer, Walter, Lehrbuch der Organischen Chemie, 21. Auflage, S. Hirzel Verlag Stuttgart 1988, Seite 213). An substituierten cyclischen Ketonen verläuft diese Reaktion mit sterisch wenig anspruchsvollen Resten jedoch nicht stereoselektiv.

Geschützte Cyanhydrine werden bei der Umsetzung von Ketonen mit Trimethylsilylcyanid erhalten (siehe die oben zitierte Literaturstelle, Beyer, Walter S. 535 ff., Hiyama, Saito, Synthesis 1985, 645-647). Bekannt ist auch die Verwendung von Oxynitrilasen zur Herstellung optisch aktiver Cyanhydrine (siehe z.B. EP-A-0 799 894, DE-A-4 102 327, WO-A-93 11255).

Aufgabe der vorliegenden Erfindung war es, ein Verfahren zur stereoselektiveren Herstellung von substituierten Cyclohexylcyanhydrinen bereitzustellen.

Gegenstand der vorliegenden Erfindung ist ein Verfahren zur stereoselektiven Herstellung von Cyclohexylcyanhydrinen der Formel (1) 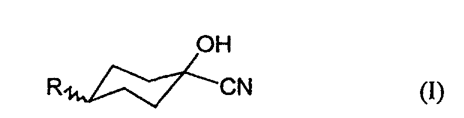 worin
- R: für jeweils gegebenenfalls substituiertes Alkyl, Cycloalkyl (worin gegebenenfalls eine Methylengruppe durch Sauerstoff ersetzt ist), Alkoxy, Alkenyloxy, Cycloalkyloxy, Arylalkyloxy, Aryloxy oder Aryl steht,
welches dadurch gekennzeichnet ist, daß man Cyclohexanone der Formel (II) 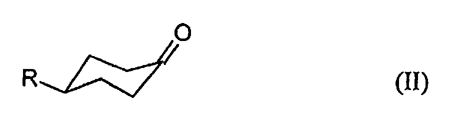 worin
- R: die obengenannten Bedeutungen hat,
in Gegenwart einer Oxynitrilase und gegebenenfalls in Gegenwart eines Verdünnungsmittels mit einer Cyanidquelle umsetzt.

Die Schlangenlinie in der Formel (I) bedeutet, daß es sich bei den Verbindungen der Formel (I) um Gemische von cis- und trans-Isomeren folgender Struktur handelt:

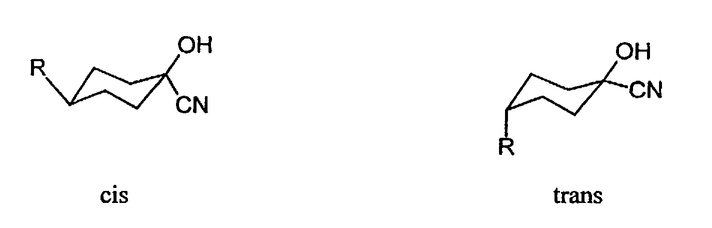

Überraschenderweise werden die Verbindungen der Formel (I) in guter Ausbeute erhalten. Umsetzungen von Cycloalkanonen mit Oxynitrilasen zu Cyanhydrinen waren nicht bekannt.

Weiterhin verläuft das erfindungsgemäße Verfahren schneller als das rein chemische Verfahren.

Das erfindungsgemäße Verfahren liefert ferner überraschenderweise in Abhängigkeit von der eingesetzten Oxynitrilase einen Überschuß an gewünschten Isomeren (cis oder trans).

Das erfindungsgemäße Verfahren läßt sich durch das folgende Formelschema wiedergeben, wenn als Cyanidquelle Blausäure verwendet wird:

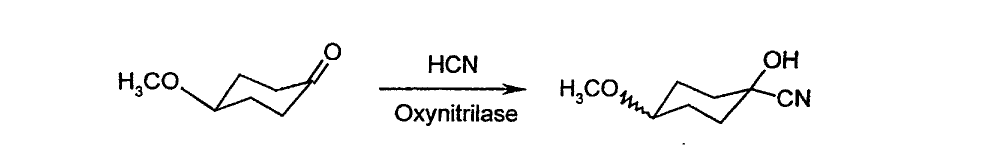

Die als Ausgangsstoffe für das erfindungsgemäße Verfahren benötigten Verbindungen sind durch die Formel (II) allgemein definiert.

Bevorzugte Substituenten bzw. Bereiche des in den oben und nachstehend erwähnten Formeln aufgeführten Restes R werden im folgenden erläutert.

Bevorzugt werden in das erfindungsgemäße Verfahren Verbindungen der Formel (II) eingesetzt, worin
- R: für gegebenenfalls durch Halogen substituiertes C₁-C₈-Alkyl, für gegebenenfalls durch C₁-C₆-Alkyl oder C₁-C₆-Alkoxy substituiertes C₃-C₈-Cycloalkyl (worin gegebenenfalls eine Methylengruppe durch Sauerstoff ersetzt ist), für gegebenenfalls durch Halogen substituiertes C₁-C₈-Alkoxy, für gegebenenfalls durch Halogen substituiertes C₃-C₆-Alkenyloxy, für gegebenenfalls durch C₁-C₆-Alkyl oder C₁-C₆-Alkoxy substituiertes C₂-C₈-Cycloalkyloxy oder für jeweils gegebenenfalls durch Halogen, C₁-C₆-Alkyl, C₁-C₆-Halogenalkyl, C₁-C₆-Alkoxy oder C₁-C₆-Halogenalkoxy substituiertes Phenyl, Phenoxy oder Benzyloxy steht.

Besonders bevorzugt werden Verbindungen der Formel (II) eingesetzt, worin
- R: für gegebenenfalls durch Halogen substituiertes C₁-C₆-Alkyl, für gegebenenfalls durch C₁-C₄-Alkyl oder C₁-C₄-Alkoxy substituiertes C₃-C₆-Cycloalkyl (worin gegebenenfalls eine Methylengruppe durch Sauerstoff ersetzt ist), für gegebenenfalls durch Halogen substituiertes C₁-C₆-Alkoxy, für gegebenenfalls durch Halogen substituiertes C₃-C₆-Alkenyloxy, für gegebenenfalls durch C₁-C₄-Alkyl oder C₁-C₄-Alkoxy substituiertes C₃-C₆-Cycloalkyloxy oder für jeweils gegebenenfalls durch Halogen, C₁-C₄-Alkyl, C₁-C₂-Halogenalkyl, C₁-C₄-Alkoxy oder C₁-C₂-Halogenalkoxy substituiertes Phenyl, Phenoxy oder Benzyloxy steht.

Halogen steht in den obigen Definitionen für Fluor, Chlor, Brom und lod, besonders für Fluor, Chlor und Brom, insbesondere für Fluor und Chlor.

Ganz besonders bevorzugt werden Verbindungen der Formel (II) eingesetzt, worin
- R: für Methyl, Ethyl, n-Propyl, i-Propyl, n-Butyl, i-Butyl, tert.-Butyl, Cyclopentyl, Cyclohexyl, Allyloxy, 2-Butenyloxy, Methoxy, Ethoxy, n-Propoxy, iso-Propoxy, n-Butoxy, iso-Butoxy, tert.-Butoxy, Phenyl oder Benzyloxy steht.

Insbesondere seien die folgenden Verbindungen der Formel (II) genannt:

| | |
|---|---|
| 4-Methylcyclohexanon | 4-Methoxycyclohexanon |
| 4-Ethylcyclohexanon | 4-Ethoxycyclohexanon |
| 4-n-Propylcyclohexanon | 4-n-Propoxycyclohexanon |
| 4-iso-Propylcyclohexanon | 4-iso-Propoxycyclohexanon |
| 4-n-Butylcyclohexanon | 4-n-Butoxycyclohexanon |
| 4-iso-Butylcyclohexanon | 4-iso-Butoxycyclohexanon |
| 4-tert.-Butylcyclohexanon | 4-tert.-Butoxycyclohexanon |
| 4-Cyclohexylcyclohexanon | 4-Allyloxycyclohexanon |
| 4-Phenylcyclohexanon | 4-Benzyloxycyclohexanon |

Die erfindungsgemäß hergestellten Verbindungen der Formel (I) sind teilweise bekannt oder lassen sich nach den in der eingangs zitierten Literatur beschriebenen Verfahren herstellen (M. Mousseron et. Al.; Bull. Soc. Chim. Fr. 4. 1435-39 (1970); P. Geneste et. al.; Bull. Soc. Chim. Fr., II, 187-191 (1980)).

Die für die Durchführung des erfindungsgemäßen Verfahrens als Ausgangsstoffe benötigten Verbindungen der Formel (II) sind teilweise bekannt oder lassen sich nach in Prinzip bekannten Verfahren herstellen (vergleiche Beispiel II-1 und z.B. J. Org. Chem. 47, 3881, 1982).

Das erfindungsgemäße Verfahren wird vorzugsweise in Gegenwart eines gegenüber den Reaktionsteilnehmern inerten Verdünnungsmittels durchgeführt.

Vorzugsweise verwendbar sind aliphatische oder aromatische Kohlenwasserstoffe, wie Benzin, Toluol, Xylol und Tetralin, ferner aliphatische oder aromatische Halogenkohlenwasserstoffe, wie Methylenchlorid, Chloroform, Tetrachlorkohlenstoff, Chlorbenzol und o-Dichlorbenzol, weiterhin offenkettige oder cyclische Ether, wie Diethylether, Methyl-tert.-butylether, Diisopropylether, Tetrahydrofuran und Dioxan, ferner Carbonsäureester, wie Essigsäureethylester, und auch stark polare Solventien, wie Dimethylsulfoxid und Sulfolan, oder auch Alkohole wie Methanol, Ethanol, Isopropanol und tert.-Butanol.

Bevorzugte Verdünnungsmittel sind Ether (insbesondere Diisopropylether) und Carbonsäureester (insbesondere Essigsäureethylester).

Die erfindungsgemäße Umsetzung kann auch in einem Zweiphasensystem durchgeführt werden, das aus Wasser und einem mit Wasser nicht vollständig mischbaren organischen Lösungsmittel besteht. Als organische Lösungsmittel kommen hierfür beispielsweise Methyl-tert.-butylether, Diisopropylether und Essigsäureethylester in Frage. Bevorzugt wird Diisopropylether verwendet.

Das erfindungsgemäße Verfahren läßt sich auch in einem wäßrigen System in Abwesenheit eines organischen Lösungsmittels bei einem bevorzugten pH <5, insbesondere bei einem pH-Wert zwischen 3 und 4 durchführen. Der pH-Wert wird durch einen Puffer eingestellt. Geeignete Puffer sind beispielsweise Natriumacetat oder Natriumcitrat. Bevorzugt wird ein 20 bis 500 mM Natriumcitrat-Puffer verwendet.

Das erfindungsgemäße Verfahren wird in Gegenwart einer Oxynitrilase (Hydroxynitril- Lyase) durchgeführt. Oxynitrilasen sind Enzyme, die die Spaltung und Bildung von Cyanhydrinen katalysieren. Oxynitrilasen sind lange bekannt (siehe z.B. EP-A-0 799 894; Chem. Commun. 1997, 1933; Angew. Chem. 1994, 106, 1609-1619; Angew. Chem. Int. Ed. Engl. 1996, 35, 438; Enantiomer, Vol. 1, pp. 359-363).

Für das erfindungsgemäße Verfahren werden bevorzugt die folgenden Oxynitrilasen eingesetzt: (R)-Oxynitrilase aus der Bittermandel (Prunus amygdalus), (S)-Oxynitrilase aus Maniok (Manihot esculenta) und (S)-Oxynitrilase aus dem Gummibaum (Hevea brasiliensis). Besonders bevorzugt werden die (R)-Oxynitrilase aus der Bittermandel (Prunus amygdalus) und die (S)-Oxynitrilase aus Maniok (Manihot exculenta) eingesetzt. Ganz besonders bevorzugt wird die (R)-Oxynitrilase aus der Bittermandel (Prunus amygdalus) verwendet.

Das erfindungsgemäße Verfahren führt in Abhängigkeit von der Art der eingesetzten Oxynitrilase zu einem Überschuß an trans- bzw. cis-Isomeren. In einigen Fällen ist darüber hinaus eine Abhängigkeit des Reaktionsverlaufs von der Art des Substituenten R zu beobachten.

Der Einsatz der (R)-Oxynitriliase aus Bittermandel führt grundsätzlich zu einem Überschuß an trans-Isomeren.

Bei Verwendung der (S)-Oxynitrilase aus Maniok überwiegt für die kleinen Reste R Methyl und Methoxy überraschenderweise das trans-Isomer, während für größere Reste R regelmäßig ein Überschuß des cis-Isomeren erhalten wird.

Die Verwendung der (S)-Oxynitrilase aus dem Gummibaum liefert bei kleineren Resten R (beispielsweise Methoxy) ebenfalls einen Überschuß an trans-Isomeren.

Die Gewinnung der genannten Oxynitrilasen ist in der Literatur beschrieben.

Für die Isolierung der (R)-Oxynitrilase [EC 4.1.2.10] aus Bittermandeln siehe z.B. Biochem. Z. 1963, 337, 156-166 und 1966, 346, 301-321; Proteins 1994, 19, 343-347; Helv. Chim. Acta, 1983, 66, 489. Für die Isolierung der (S)-Oxynitrilase [EC 4.1.2.37] aus Maniok siehe z.B. F.J.P. De C. Carvalho, Dissertation, University of California, Davis, 1981; Arch. Biochem. Biophys. 1994, 311, 496-502. Für die Isolierung der (S)-Oxynitrilase aus dem Gummibaum siehe z.B. Physiologia Plantarum 1989, 75, 97; Plant Science 1996, 115, 25-31.

Grundsätzlich können die Oxynitrilasen in verschiedenen Aufbereitungsformen in das erfindungsgemäße Verfahren eingesetzt werden.

Andererseits kann auch Rohenzym verwendet werden, d.h., das Enzym wird nicht isoliert, sondern es wird ein zellfreier Extrakt eingesetzt, den man erhält, indem man die Zellen mit Ultraschall aufschließt, den so gewonnenen Rohextrakt von den Zellwänden/-membranen abzentrifugiert und aufkonzentriert. Literatur z.B. (R)-Oxynitrilase: Tetrah. Lett. 1988, 29, 4485-88, Tetrah. Ass. 1996, 7, 1105-1116, Synthetic Communications 1991, 21, 1387-91.

Für die Verwendung von (R)-Oxynitrilen aus Bittermandeln eignet sich auch der Einsatz von vorzugsweise entfettetem Mandelmehl, das man erhält, indem man Bittermandeln mahlt und mit z.B. Hexan oder Petrolehter extrahiert (käuflich bei Sigma) oder Lit.: Lett., 1988, 29, 4485-4488.

### Bevorzugt wird gereinigtes Enzym verwendet

Die Enzyme können für die Durchführung der erfindungsgemäßen Umsetzung an geeignetes Trägermaterial gebunden werden. Als Trägermaterial kommen beispielsweise Cellulosen in Frage, insbesondere Nitrocellulose und Cellulose P 100 PSC: Elcema®; Degussa, Teilchengröße 50-150 µm, an der Oberfläche mit 2 % amorpher Kieselsäure (Aerosil) überzogen. Diese Verfahrensweise ermöglicht nach beendeter Reaktion die Abfiltrierung von trägergebundenem Enzym und damit eine vereinfachte Aufarbeitung. Das so zurückgewonnene trägergebundene Enzym kann darüber hinaus erneut verwendet werden.

Das Enzym wird in der Regel auf den Träger aufgebracht, indem man die Enzymlösung auf den Träger tropft, siehe z.B. auch Angew. Chem. Int. Ed. Engl. 1996, 35, 439.

Das erfindungsgemäße Verfahren wird in Gegenwart einer Cyanidquelle durchgeführt.

Als Cyanidquelle kommen alle Verbindungen in Frage, die Cyanidionen (CN⁻) freisetzen. Beispielsweise seien Blausäure, Cyanhydrine wie Aceton-cyanhydrin und Metallcyanide wie KCN genannt. Bevorzugt wird Blausäure oder KCN, besonders bevorzugt Blausäure verwendet.

Die Temperatur kann bei dem erfindungsgemäßen Verfahren in einem größeren Bereich variiert werden. Im allgemeinen arbeitet man bei Temperaturen zwischen -20°C und 80°C, bevorzugt zwischen 0°C und 40°C.

Das erfindungsgemäße Verfahren wird im allgemeinen unter Normaldruck durchgeführt.

Das molare Verhältnis an Verbindung der Formel (II) zu Cyanidionen liegt im allgemeinen zwischen 1:1 und 1:50, bevorzugt zwischen 1:1 und 1:4.

Im allgemeinen geht man bei der Durchführung des erfindungsgemäßen Verfahrens so vor, daß man die Verbindung der Formel (II) und die Cyanidquelle in einem geeigneten Verdünnungsmittel vorlegt, das immobilisierte Enzym zugibt und das Reaktionsgemisch bis zur beendeten Reaktion bei Raumtemperatur rührt. Anschließend wird der Katalysator abfiltriert und das Filtrat mit üblichen Methoden aufgearbeitet.

Grundsätzlich ist es auch möglich, die Umsetzung kontinuierlich durchzuführen, beispielsweise, indem man die Edukte im Verdünnungsmittel gelöst über eine Säule schickt, die mit dem trägerfixierten Enzym beladen ist oder in einem Membranreaktor reagieren läßt, wobei die Produkte von den Edukten und dem Katalysator durch Evaporation abgetrennt werden.

Die mit dem erfindungsgemäßen Verfahren hergestellten Verbindungen der Formel (I) sind wertvolle Zwischenprodukte für die Herstellung von Herbiziden und Schädlingsbekämpfungsmitteln gemäß folgendem Reaktionsschema: (siehe z.B. EP 0 647 637).

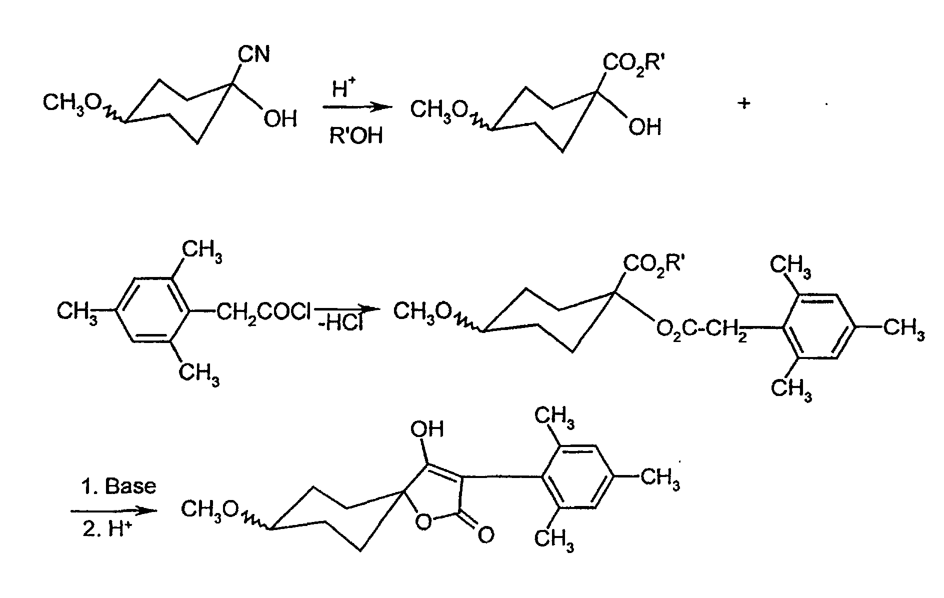

### Beispiele

### Beispiel 1 (Substratkonzentration 0,2 mol/l)

Zu einer Lösung von 1 mmol Keton und 3,9 mmol Blausäure in 5 ml Diisopropylether wurde das auf Cellulose *(Prunus amygdalus)* bzw. Nitro-cellulose immobilisierte Enzym zugegeben und die angegebene Zeit bei Raumtemperatur gerührt.

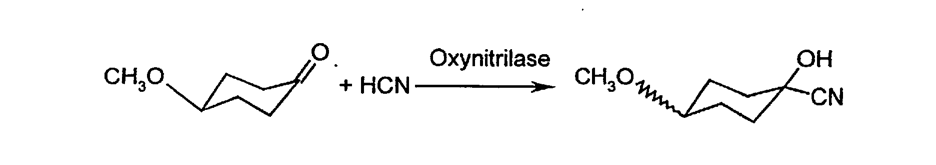

| Enzym aus | Units/mmol | Reaktions dauer (h) | Umsatz* (%) | Isomerenverhältnis* (trans:cis) |
|---|---|---|---|---|
| a) Prunus amygdalus | 100 | 4,5 | 92 | 92:8 |
| b) Manihot esculenta | 100 | 1 | quant. | 70:30 |
| c) Hevea brasiliensis | 1000** | 2 | quant. | 65:35 |

| | | | | |
|---|---|---|---|---|
| * Die nicht enzymkatalysierte Konkurrenzreaktion fällt hier nicht ins Gewicht: sie erreicht lediglich 6 % Umsatz in 10 Stunden mit einem Isomerenverhältnis von 54:46 (cis:trans) | | | | |
| ** Aktivitätstest mit (S)-Mandelonitril als Substrat | | | | |

Die Enzyme wurden nach folgenden Vorschriften erhalten bzw. auf die Träger aufgebracht:
Die Enzymlösung der (R)-Oxynitrilase aus *Prunus amygdalus* gibt man auf den Celluloseträger, der zuvor in einem Natriumcitrat-Puffer (20mM, pH 3,3) gequollen und anschließend abgepreßt wurde. Den enzymbeladenen Träger verwendet man so direkt
Die Enzymlösungen der beiden (S)-Oxynitrilasen gibt man auf Nitrocellulose, die zuvor ebenfalls in einem Natriumcitrat-Puffer (20mM, pH 3,3) gequollen wurde und anschließend im Hochvakuum getrocknet wurde. Den enzymbeladenen Träger zentrifugiert man, um das überschüssige Wasser zu entfernen (Angew. Chem. Int. Ed. Eng. 1996 35, No. 4,437-439).

Es wurden in den folgenden Beispielen 2 bis 5 (R)-Oxynitrilase aus der Bittermandel verwendet (100 U des Enzyms pro 1 mmol Keton) und folgende Reaktion durchgeführt:

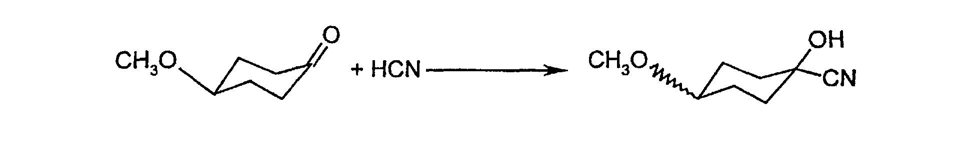

### Beispiel 2 (Substratkonzentration 0,2 mol/l)

| Lösungsmittel | Reaktionsdauer (h) | Umsatz (%) | Isomerenverhältnis (trans:cis) |
|---|---|---|---|
| a) Diisopropylether | 4,5 | 92 | 92:8 |
| b) tert.-Butylmethylether | 23 | 82 | 87:13 |
| c) Essigsäureethylester | 24 | 17 | 74:26 |

### Beispiel 3 (Substratkonzentration 0,2 mol/l)

Bei der Variation des Trägermaterials stand vor allem die Absenkung des Wassergehaltes auf dem Träger im Vordergrund. Durch die Zentrifugation der mit Enzymlösung beladenen Nitrocellulose gelingt es, den Wassergehalt sehr stark zu senken.

| Träger | Reaktionsdauer (h) | Umsatz (%) | Isomerenverhältnis (trans:cis). |
|---|---|---|---|
| a) Cellulose P100PSC | 4,5 | 92 | 92:8 |
| b) Nitrocellulose | 7 | 62 | 91:9 |

### Beispiel 4 (Substratkonzentration 0,2 mol/l)

| Reaktionstemperatur (°C) | Reaktionsdauer (h) | Umsatz (%) | Isomerenverhältnis (trans:cis) |
|---|---|---|---|
| a) 0 | 1 | 41 | 91:9 |
| b) 25 | 2 | 74 | 91:9 |
| c) 42 | 2 | quant. | 89:11 |

### Beispiel 5

| Substratkonz [M] | Reaktionsdauer (h) | Umsatz (%) | Isomerenverhältnis (trans:cis) |
|---|---|---|---|
| a) 0,2 | 4,5 | 92 | 92:8 |
| b) 0,6 | 2 | quant. | 90:10 |
| c) 1,0 | 2 | 87 | 82:18 |

### Beispiel 6

### (R)-Oxynitrilase-katalysierte Addition von Blausäure an Cyclohexanonderivate

Alle Umsetzungen wurden in Diisopropylether und bei Raumtemperatur durchgeführt. Die (R)-Oxynitrilase wurde auf Cellulose adsorbiert, die zuvor in 20 mM Citratpufferlösung pH 3,3 gequollen wurde. Blausäure wurde im vierfachen Überschuß zum Substrat zugegeben. Zu jedem Enzymansatz wurde eine Blindprobe durchgeführt, um die chemische Reaktion abschätzen zu können.

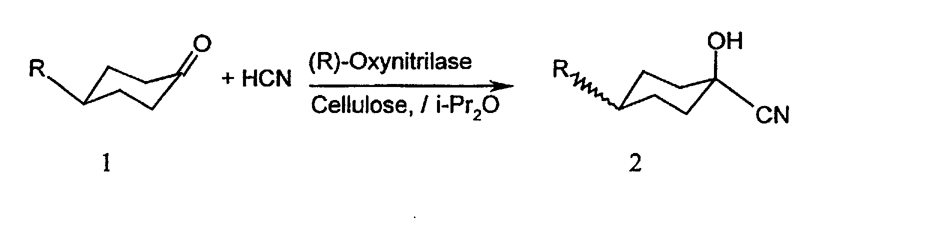

**Tabelle l**

| | | Cyanhydrin 2 | | | Blindprobe [b] | |
|---|---|---|---|---|---|---|
| R | U/mmol | t [h] | Umsatz [%] | cis/trans [a][%] | Umsatz [%] | cis/trans [a] [%] |
| Methyl- | 200 | 1,5 | 99 | 3:97 | 4 | 17:83 |
| Ethyl- | 200 | 7,25 | 92 | 2:98 | <0,5 | n.b. [c] |
| Propyl- | 200 | 22 | 73 | 2:98 | <1 | n.b. |
| *iso*Propyl- | 200 | 22 | 94 | 1:99 | <1 | n.b. |
| tert.-Butyl- | 200 | 216 | 50 | 10:90 | 2 | 13:87 |
| Ethoxy- | 200 | 24 | 99 | 11:89 | 78 | 43:57 |
| Propoxy | 200 | 48 | 60 | 14:86 | 21 | 46:54 |
| Phenyl- | 200 | 360 | 83 | 5:95 | 49 | 10:90 |

| | | | | | | |
|---|---|---|---|---|---|---|
| [a] Isomerenverhältnis nach Acetylierung mit Dimethylaminopyridin und Acetanhydrid gaschromatographisch bestimmt. | | | | | | |
| [b] Anstatt der Enzymlösung wurde eine entsprechende Menge eines 20 mM Natriumacetat-Puffers pH 5,4 verwendet. | | | | | | |
| [c] Nicht bestimmt. | | | | | | |

### Beispiel 7

### (S)-Oxynitrilase-katalysierte Addition von Blausäure an Cyclohexanonderivate

Für die Umsetzungen von (S)-Oxynitrilase aus Maniak (Manihot esculenta) wurden bis auf Nitrocellulose als neues Enzymträgermaterial die gleichen optimierten Reaktionsbedingungen gewählt wie für die (R)-Oxynitrilase aus Bittermandeln.

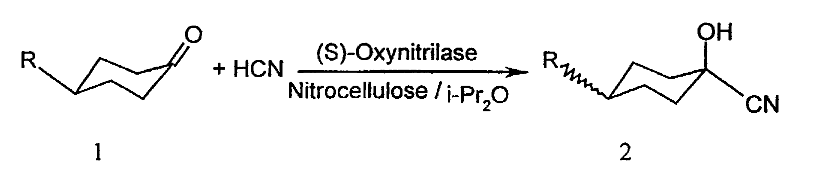

**Tabelle 2**

| | | | Cyanhydrin 2 | | Blindprobe | |
|---|---|---|---|---|---|---|
| R | U/mmol | t [h] | Umsatz [%] | IV cis/trans [a] [%] | Umsatz [%] | cis/trans [%] |
| Methyl- | 100 | 2 | 99 | 35:65 | | |
| Ethyl- | 100 | 3 | 93 | 74:26 | <0,5 | n.b. [b] |
| Propyl- | 100 | 5 | 96 | 96:4 | <1 | n.b. |
| *iso*Propyl- | 100 | 5 | 93 | 97:3 | <1 | n.b. |
| *tert*.-Butyl | 100 | 3 | 82 | 99:1 | | |
| Ethoxy- | 100 | 1 | 99 | 61:39 | | |
| Propoxy- | 100 | 2 | 91 | 87:13 | | |
| Phenyl- | 100 | 3 | 95 | 99:1 | | |

| | | | | | | |
|---|---|---|---|---|---|---|
| [a] Isomerenverhältnis nach Acetylierung mit Dimethylaminopyridin und Acetanhydrid gaschromatographisch bestimmt. | | | | | | |
| [b] Nicht bestimmt | | | | | | |

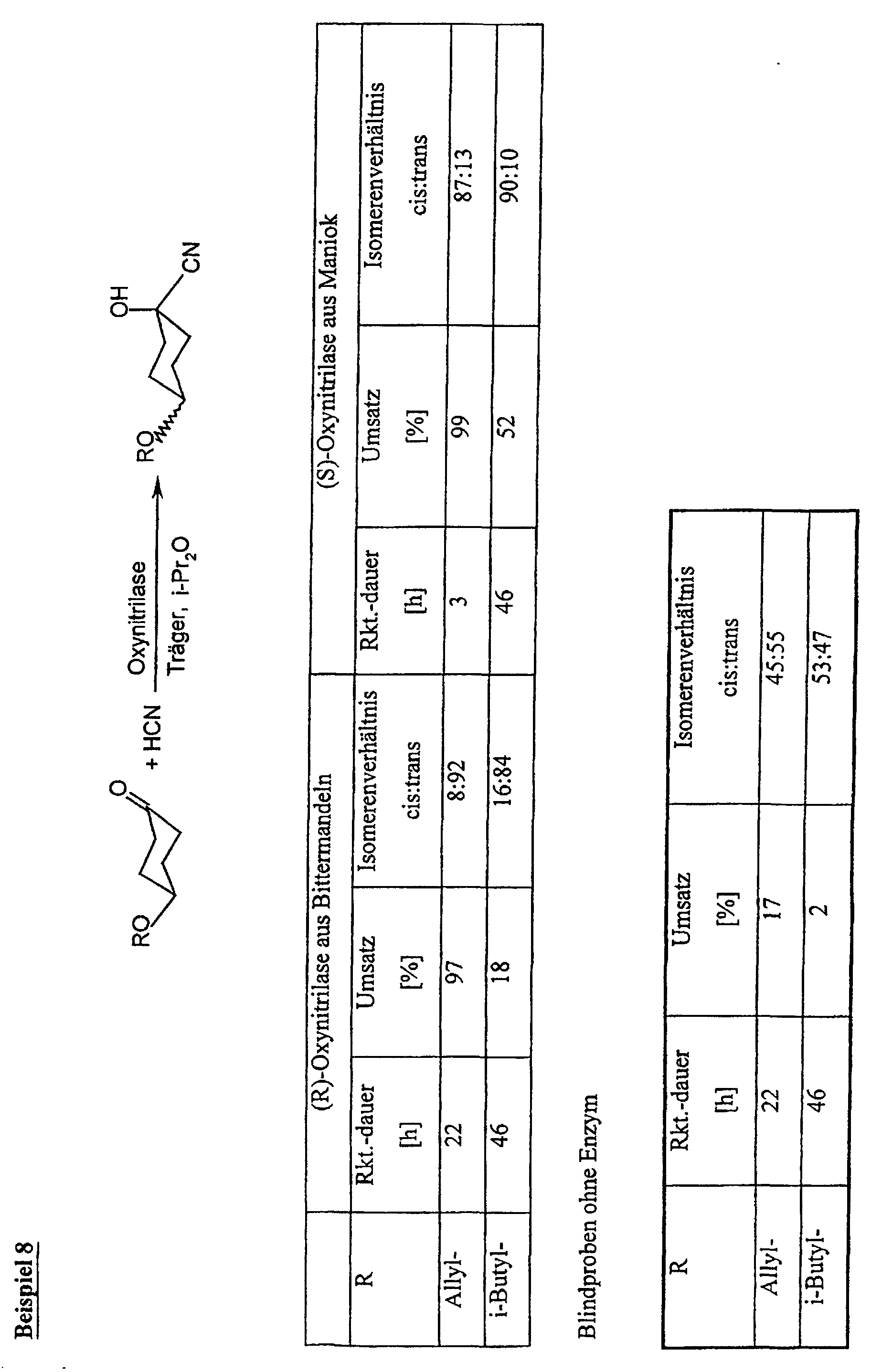

### Beispiel (II-l)

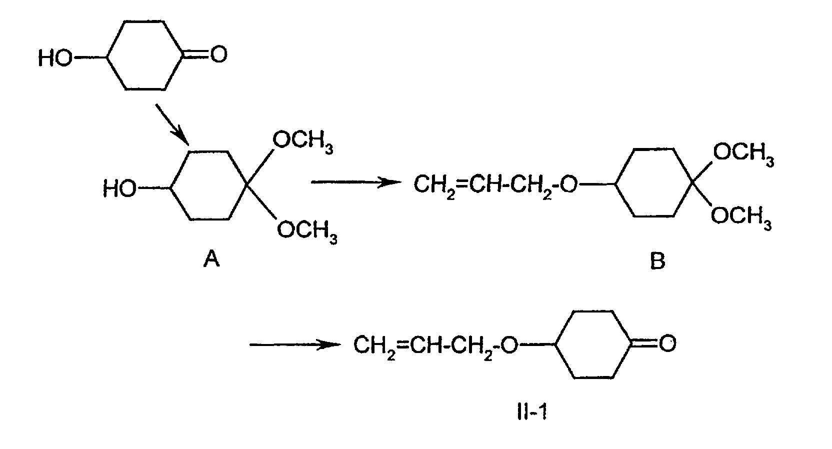

### Herstellung der Verbindung A

94 g käufliches 4-Hydroxycyclohexanon in 275 ml ortho-Ameisensäuretrimethylester werden bei Raumtemperatur mit 260 g Methanol und 5 mg para-Toluolsulfonsäure in 20 ml Methanol versetzt und 10 Minuten unter Rückfluß gekocht. Dann wird bei ca. 0°C mit wässriger Natriumhydrogencarbonatlösung neutralisiert und eingeengt. Der Rückstand wird an Kieselgel mit dem Laufmittel Hexan/Aceton 7/3 chromatographiert.

Ausbeute: 125 g (95 % der Theorie).

### Herstellung der Verbindung B

Zu 200 g der Verbindung A in 1500 ml tert.-Butanol gibt man bei Raumtemperatur 150 g Kalium-tert.-butylat und 151 g Allylbromid und rührt einen Tag bei ca. 60°C. Dann wird mit Wasser verdünnt, mit Methylenchlorid extrahiert und die organische Phase eingeengt. Der Rückstand wird an Kieselgel mit dem Laufmittel Hexan/Aceton 10/1 chromatographiert.

Ausbeute: 215 g (86 % der Theorie).

### Herstellung der Verbindung (II-1)

233 g der Verbindung 3 werden in 1000 ml Tetrahydrofuran (THF) und 750ml IN HCl 2 Stunden bei Raumtemperatur gerührt. Dann wird mit Wasser versetzt und mit Methylenchlorid extrahiert. Die organische Phase wird eingeengt.

Ausbeute: 157 g (88 % der Theorie).

## Patentansprüche

1. Verfahren zur stereoselektiven Herstellung von Cyclohexylcyanhydrinen der Formel (I) 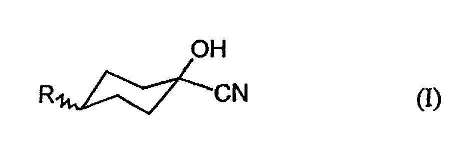 worin
R für jeweils gegebenenfalls substituiertes Alkyl, Cycloalkyl (worin gegebenenfalls eine Methylengruppe durch Sauerstoff ersetzt ist), Alkoxy, Alkenyloxy, Cycloalkyloxy, Arylalkyloxy oder Aryl steht,
**dadurch gekennzeichnet, daß** man Cyclohexanone der Formel (II) 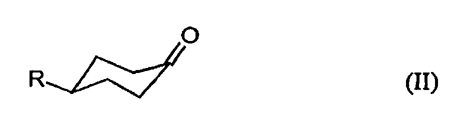 worin
R die obengenannten Bedeutungen hat,
in Gegenwart einer Oxynitrilase und gegebenenfalls in Gegenwart eines Verdünnungsmittels mit einer Cyanidquelle umsetzt.

2. Verfahren zur Herstellung von trans-Cyclohexylcyanhydrinen der Formel (Ib) 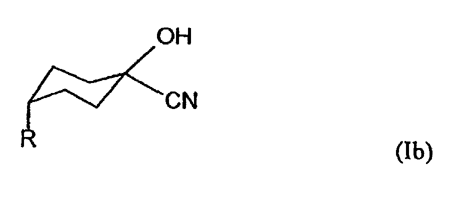 worin
R die in Anspruch 1 angegebenen Bedeutungen hat, durch Umsetzung von Cylcohexanonen der Formel (II) 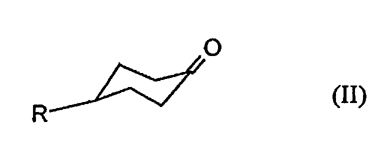
mit einer Cyanidquelle in Gegenwart von (R)-Oxynitrilase aus Bittermandel und gegebenenfalls in Gegenwart eines Verdünnungsmittels.

## Claims

1. Process for the stereoselective preparation of cyclohexylcyanohydrins of the formula (I) 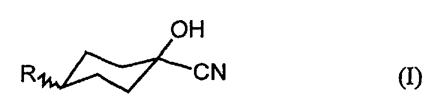 in which
R represents alkyl, cycloalkyl (in which a methylene group is optionally replaced by oxygen), alkoxy, alkenyloxy, cycloalkyloxy, arylalkyloxy or aryl, in each case optionally substituted,
**characterized in that** cyclohexanones of the formula (II) 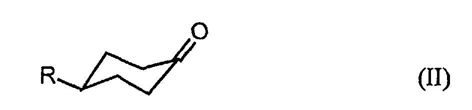 in which
R has the abovementioned meanings,
are reacted with a cyanide source in the presence of an oxynitrilase and if appropriate in the presence of a diluent.

2. Process for the preparation of trans-cyclohexylcyanohydrins of the formula (Ib) 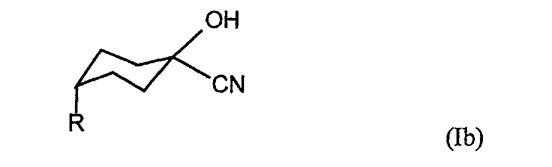 in which
R has the meanings indicated in Claim 1, by reaction of cyclohexanones of the formula (II) 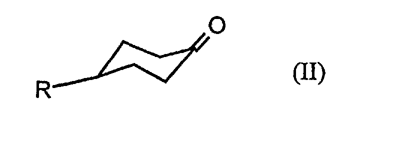
with a cyanide source in the presence of (R)-oxynitrilase from bitter almond and if appropriate in the presence of a diluent.

## Revendications

1. Procédé de préparation stéréosélective de cyclohexylcyanhydrines de formule (I) : 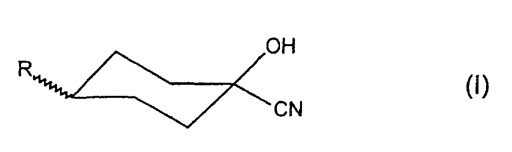 où
R représente un radical, chaque fois le cas échéant substitué, alcoyle, cycloalcoyle (où le cas échéant, un radical méthylène peut être remplacé par l'atome d'oxygène), alcoxy, alcényloxy, cycloalcoyloxy, arylalcoyloxy ou aryle,
qui est **caractérisé en ce que** l'on fait réagir une cyclohexanone de formule (II) : 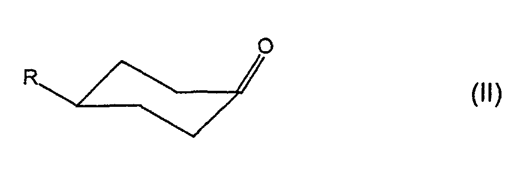 où
R a les significations indiquées ci-dessus,
en présence d'une oxynitrilase et le cas échéant, en présence d'un agent de dilution avec une source de cyanure.

2. Procédé de préparation de trans-cyclohexylcyanhydrines de formule (Ib) : 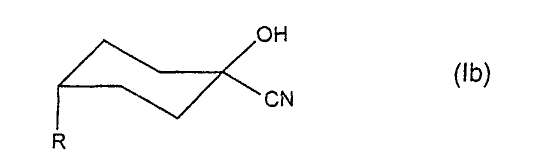 où
R a la signification indiquée à la revendication 1, par réaction de la cyclohexanone de formule (II) : 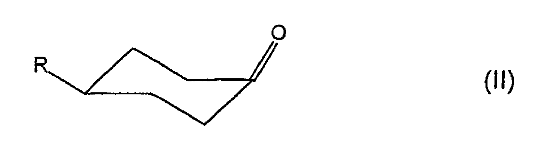 avec une source de cyanure en présence de la (R)-oxynitrilase d'amandier et le cas échéant, en présence d'un agent de dilution.
